(19) Europäisches Patentamt
European Patent Office
Office européen des brevets

(11) Veröffentlichungsnummer: **0 079 311**
**B1**

(12) # EUROPÄISCHE PATENTSCHRIFT

(45) Veröffentlichungstag der Patentschrift: **18.03.87**

(51) Int. Cl.[4]: **C 07 D 213/64,** A 01 N 47/34

(21) Anmeldenummer: **82810470.3**

(22) Anmeldetag: **04.11.82**

(54) **Phenylbenzoylharnstoffe zur Bekämpfung von Schädlingen.**

(30) Priorität: **10.11.81 CH 7208/81**
**08.10.82 CH 5925/82**

(43) Veröffentlichungstag der Anmeldung:
**18.05.83 Patentblatt 83/20**

(45) Bekanntmachung des Hinweises auf die Patenterteilung:
**18.03.87 Patentblatt 87/12**

(84) Benannte Vertragsstaaten:
**AT CH DE FR IT LI NL**

(56) Entgegenhaltungen:
**EP-A-0 025 363**
**EP-A-0 040 179**
**DE-A-2 818 830**
**US-A-4 003 733**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Böger, Manfred, Wilhelm Glock- Strasse 14, D-7858 Weil am Rhein 5 (DE)**

**EP 0 079 311 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue N-3-(5-Trifluormethyl-pyridyl-2-oxy)-phenyl-N'-benzoylharnstoffe, Verfahren zu ihrer Herstellung und ihre Verwendung in der Schädlingsbekämpfung. Neue Ausgangsstoffe und deren Herstellung bilden ebenfalls einen Gegenstand der Erfindung.

Die erfindungsgemässen substituierten N-3-(5-Trifluormethyl-pyridyl-2-oxy)-phenyl-N'-benzoylharnstoffe haben die Formel I

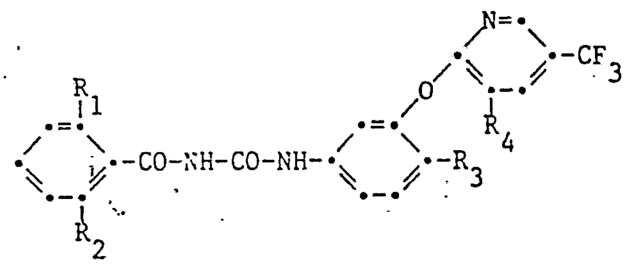

(I),

worin

$R_1$, $R_2$ und $R_3$ unabhängig voneinander Wasserstoff, Methyl, Fluor, Chlor oder Brom; und

$R_4$ Wasserstoff oder Chlor bedeuten, mit der Massgabe, dass $R_3$ Methyl, Fluor, Chlor oder Brom bedeutet, wenn $R_4$ die Bedeutung von Chlor hat.

Wegen ihrer Wirkung als Schädlingsbekämpfungsmittel bevorzugt sind Verbindungen der Formel I, worin $R_1$ Methyl, Fluor oder Chlor, $R_2$ Wasserstoff, Methyl, Fluor oder Chlor, $R_3$ Wasserstoff, Methyl, Fluor, Chlor oder Brom und $R_4$ Wasserstoff oder Chlor bedeuten. Von besonderen Interesse sind ferner solche Verbindungen der Formel I, worin $R_1$ Fluor oder Chlor, $R_2$ Wasserstoff oder Fluor, $R_3$ Wasserstoff, Methyl oder Chlor und $R_4$ Wasserstoff oder Chlor bedeuten.

Weiterhin bevorzugt werden Verbindungen der Formel I, worin $R_3$ und/ oder $R_4$ Wasserstoff bedeuten. Hervorzuheben sind insbesondere solche Verbindungen der Formel I, worin $R_1$ und $R_2$ Fluor bedeuten.

Die Verbindungen der Formel I können analog an sich bekannter Verfahren hergestellt werden (vgl. u.a. die deutschen Offenlegungsschriften Nr. 2.123.236, 2.601.780).

So kann man z.B. eine Verbindung der Formel I erhalten, durch Umsetzung

a) einer Verbindung der Formel II

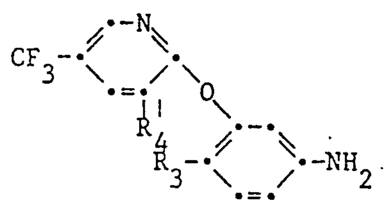

(II)

mit einer Verbindung der Formel III

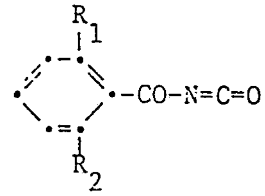

(III)

oder
b) einer Verbindung der Formel IV

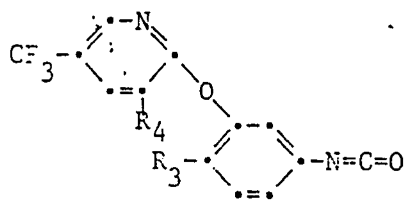

(IV)

gegebenenfalls in Gegenwart einer organischen oder anorganischen Base mit einer Verbindung der Formel V

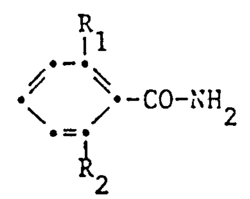

(V),

oder
c) einer Verbindung der Formel II mit einer Verbindung der Formel VI

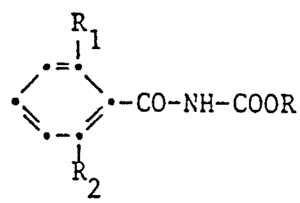

(VI).

In den obigen Formeln II bis VI haben die Reste $R_1$ bis $R_4$ die unter Formel I vorstehend angegebenen Bedeutungen und R bedeutet einen $C_1$-$C_8$-Alkylrest, der gegebenenfalls mit Halogen substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 100°C, vorzugsweise zwischen 15 und 25°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit dem Anilin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylol, Chlorbenzol usw. verwendet werden.

Die Ausgangsstoffe der Formeln III, V und VI sind bekannt und können analog bekannten Verfahren hergestellt werden. Bei den Ausgangsstoffen der Formel II und IV handelt es sich teilweise um neuartige Verbindungen, die nach an sich bekannten Arbeitsweisen hergestellt werden können (vgl. z.B. die US-Patentschriften Nr. 3.705.170 und 3.711.486).

5-Trifluormethylpyridyl-2-oxy-aniline der Formel II können wie folgt erhalten werden:

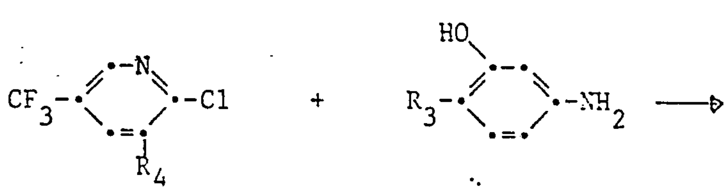

(II).

Diese Umsetzung wird bei einer Temperatur von 20-180°C, vorzugsweise 50-160°C, in Gegenwart eines Säureacceptors, z.B. eines Alkali- oder Erdalkalihydroxids oder -hydrids, vorzugsweise KOH oder NaOH, sowie eines inerten organischen Lösungsmittels, vorzugsweise Dimethylformamid oder Dimethylsulfoxid, durchgeführt. Weiterhin ist ein Anilin der Formel II in Analogie zu dem in J.Org.Chem. 29 (1964) 1, aufgezeigten Verfahren durch Hydrierung der entsprechenden Nitro-Verbindungen herstellbar (vgl. auch die dort zitierte Literatur); auch durch chemische Reduktion (z.B. mittels Sn-(II)-Chlorid/HCl) einer entsprechenden Nitroverbindung sind Aniline der Formel II zugänglich (vgl. Houben Weyl, "Methoden d. org. Chemie" 11-/1, 422):

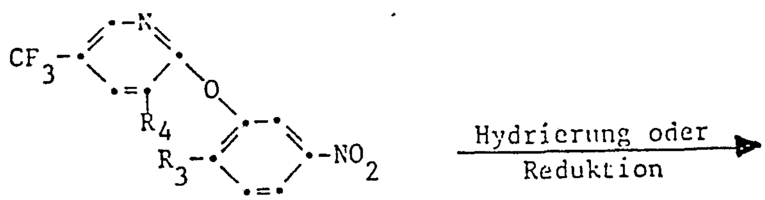


(II).

Gegenstand der vorliegenden Erfindung sind auch die neuen Verbindungen der Formel II, worin $R_4$ Wasserstoff und $R_3$ Wasserstoff, Methyl, Fluor, Chlor oder Brom bedeuten, oder worin $R_4$ Chlor und $R_3$ Methyl, Fluor, Chlor oder Brom darstellen.

Zu Benzoylisocyanaten der Formel III kann man unter anderem wie folgt gelangen (vgl. J. Agr. Food Chem. 21, 348 und 993; 1973):

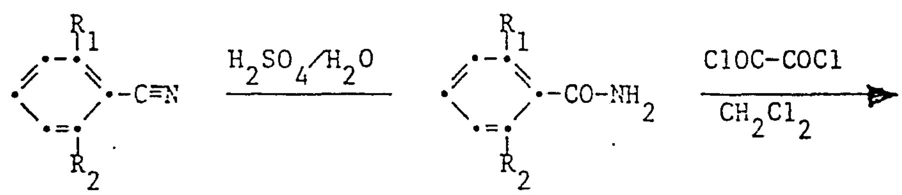


(III).

Ein 3-(5-Trifluormethylpyridyl-2-oxy)-phenylisocyanat der Formel IV lässt sich z.B. durch Phosgenierung eines Anilins der Formel II nach allgemein üblichen Verfahren herstellen. Die weiterhin als Ausgangsstoffe zu verwendenden Benzamide der Formel V sind bekannt (vgl. z.B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure.

Es ist bereits bekannt, dass bestimmte substituierte N-Phenoxyphenyl-N'-benzoylharnstoffe insektizide Eigenschaften besitzen. So sind aus den deutschen Offenlegungsschriften 2 504 982 und 2 537 413 halogensubstituierte N-4-(2-Chlor-4-trifluormethyl-phenoxy)-phenyl-N'-benzoylharnstoffe mit insektizider Wirkung bekannt. Die japanische Patentschrift JP-A-53 103 447 betrifft N-4(Trifluormethylphenoxy)-phenyl-N'-benzoylharnstoffe als insektizide Wirkstoffe. Weiterhin werden in den deutschen Offenlegungsschriften 2 748 636 und 2 818 830, in der Europäischen Patentanmeldung 25363 sowie in der japanischen Patentschrift JP-A-54 115 380 unter anderem N-4(5-Trifluormethyl-pyridyl-2-oxy)-phenyl-N'-benzoylharnstoffe mit selektiver insektizider Wirkung beschrieben, d.h. es werden nützliche Insekten geschont und Schadinsekten bekämpft.

Ferner werden in der EP-A-40179 strukturell ähnliche, N-3-(5-Trifluormethyl-pyridyl-2-oxy)-phenyl-N'-benzoylharnstoffe beschrieben.

Bei den erfindungsgemässen Verbindungen der Formel I handelt es sich demgegenüber um neuartige substituierte N-3-(5-Trifluormethyl-pyridyl-2-oxy)-phenyl-N'-benzoylharnstoffe, die überraschenderweise erhöhte insektizide Wirksamkeit, insbesondere gegen fressende Schadinsekten, wie Spodoptera littoralis und Heliothis virescens, aufweisen. Ein weiterer Vorteil der erfindungsgemässen Verbindungen der Formel I ergibt sich aus ihrer sehr geringen Warmblütertoxizität bei guter Pflanzenverträglichkeit.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. gegen Spodoptera littoralis und Heliothis virescens), sowie in Obst- und Gemüsekulturen (z.B. gegen Laspeyresia pomonella, Leptinotarsa decemlineata und Epilachna varivestis) eingesetzt werden. Die Verbindungen der Formel I zeichnen sich durch eine ausgeprägte Wirkung gegen larvale Insektenstadien, insbesondere larvale Stadien fressender Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weide-Behandlung.

Die bisher bekanntgewordenen N-Phenoxyphenyl-N'-benzoylharnstoffe werden in der Literatur als fast ausschliesslich insektizid wirksam beschrieben. Andere Wirkungen sind nur am Rande beobachtet worden. Eine angeblich akarizide Wirkung wird z.B. in der DE-OS 2 504 982 beiläufig erwähnt, jedoch nicht nachgewiesen.

Es ist nun besonders überraschend und konnte der vorbekannten Literatur nicht entnommen werden, dass die Verbindungen der Formel I nicht nur eine günstige insektizide, sondern darüberhinaus eine besonders ausgeprägte akarizide Wirkung besitzen, die vor allem tierparasitäre Vertreter der Ordnung Acarina erfasst. Die akarizide Verwendung der Verbindungen der Formel I ist ein Gegenstand vorliegender Erfindung. Der besondere Vorteil liegt in der Bekämpfungsmöglichkeit sämtlicher Entwicklungsstadien von Acarina, so dass eine einmal durchgeführte Applikation sowohl auf adulte Schädlinge wie auf Nymphen, Larven und Eier abtötend wirkt. Es konnte an künstlich mit Boophilus microplus-Larven infizierten Rindern nachgewiesen werden, dass sich 2 1/2-3 wochen nach Applikation weder adulte Zecken noch ihre Entwicklungsstadien auf den Tieren vorfanden und dass auch während der Folgezeit keine Neubesiedlung erfolgte. Dieser Befund ist ausserordentlich überraschend und beweist darüberhinaus die Existenz einer zusätzlichen und ganz unerwarteten neuartigen Dauerwirkung der Präparate der Formel I.

Infolge dieser ausgeprägten Doppelwirkung gegen Insekten und gegen Schädlinge der Ordnung Acarina sind Verbindungen der Formel I hervorragend geeignet, Ektoparasiten aller Art zu bekämpfen, d.h. sowohl pflanzenparasitäre wie vor allem tierparasitäre Vertreter.

Zur letztgenannten Gruppe gehören sowohl parasitäre Fliegen (und ihre Entwicklungsstadien) der Gattungen Musca, Fannia, Haematobia, Stomoxis, Glossina, Hippobosca, Lucilia, Calliphora, Phormia, Sarcophaga, Wohlfahrtia, Cochliomyia, Chrysomyia, Cordylobia, Dermatobia, Cuterebra, Gastrophilus, Oestrus, Hypoderma und andere, sowie Zecken und Milben der Gattungen Ixodes, Ripicephalus, Amblyomma, Hyalomma, Haemaphysalis, Boophilus, Ornithodorus, Argas, Dermanyssus, Ornithonyssus, Psoroptes, Chorioptes, Sarcoptes und andere.

Die erfindungsgemässe Anwendung der Wirkstoffe geschieht im Veterinärsektor in bekannter Weise durch orale Anwendung in Form von beispielsweise Tabletten, Kapseln, Lecksteinen, Bolussen, Tränken, Granulaten oder durch dermale Anwendung in Form beispielsweise des Tauchens, Sprühens, Aufgiessens ("pour-on" oder "spot-on") und des Einpuderns oder durch parenterale Anwendung in Form der Injektion oder eines Implantats, oder durch transdermale Applikation mit auf der Haut angebrachten Kapseln.

Die Wirkung der erfindungsgemässen Verbindungen bzw. der sie enthaltenden Mittel lässt sich durch Zusatz von anderen Insektiziden und/ oder Akariziden wesentlich verbreitern und an gegebene Umstände anpassen.

Als Zusätze kommen z.B. folgende Wirkstoffe in Betracht: organische Phosphorverbindungen, Nitrophenole und Derivate, Formamidin, Harnstoffe, Carbamate, chlorierte Kohlenwasserstoffe und bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a: Piperonylbutoxid, Propinyläther, Propinyloxime, Propinylcarbamate und Propinylphosphonate, 2-(3,4-Methylendioxyphenoxy)-2,6,9-trioxaundecan oder S,S,S-Tributylphosphorotrithioate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren, verdünnbaren oder parenteral injizierbaren Lösungen, verdünnten Emulsionen, Spritzpulvern, löslichen Pulvern, Stäubemitteln, Granulaten, Komprimaten, auch Verkapselungen in z.B. polymeren Stoffen in bekannter Weise verarbeitet. Die Anwendungsverfahren, wie Versprühen, Vernebeln, Verstäuben, Verstreuen, Giessen, orale Applikation oder Injektion, werden ebenso wie die Mittel den angestrebten Zielen und den gegebenen Verhältnissen entsprechend gewählt.

Die Formulierungen, d.h. die den Wirkstoff der Formel I bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone,

wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxydiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden.

Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als Hilfsstoffe für die Komprimate kommen die üblichen Bindemittel wie Dextrine, Cellulosederivate, Sorbitol und Polyvinylpyrrolidon; Füllstoffe wie Cellulose, Lactose und verschiedene Stärkearten; Gleitmittel wie Erdalkalisalze der höheren Fettsäuren z.B. Kalziumstearat; und zerfallsbeschleunigende Hilfsmittel wie Alginate, Avicel und Stärkederivate in Frage.

Als oberflächenaktive Verbindungen kommen je nach der Art des zu formulierenden Wirkstoffes der Formel I oder der Kombinationen dieser Wirkstoffe mit anderen Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen. Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood, New Jersey, 1979.

Sisely and Wood, "Encyclopedia of Surface Active Agents", Chemical Publishing Co., Inc. New York.

Die pestiziden Zubereitungen enthalten in der Regel 0,1 bis 99%, insbesondere 0,1 bis 95%, Wirkstoff der Formel I oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Formulierungsbeispiele für feste Wirkstoffe der Formel I resp. Kombinationen dieser Wirkstoffe mit anderen Insektiziden oder Akariziden (% = Gewichtsprozent)**

1. **Spritzpulver**

| | a) | b) | c) |
|---|---|---|---|
| Wirkstoff oder Wirkstoffkombination | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff oder die Wirkstoffkombination werden mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen. Diese Pulver können mit dem Futter appliziert werden.

2. **Emulsions-Konzentrat**

| | |
|---|---|
| Wirkstoff oder Wirkstoffkombination | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

3. **Stäubemittel**

| | a) | b) |
|---|---|---|
| Wirkstoff oder Wirkstoffkombination | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird. Stäubemittel können direkt appliziert oder dem Futter beigegeben werden.

4. **Extruder-Granulat**

| | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert, granuliert und anschliessend im Luftstrom getrocknet. Solche Granulate eignen sich auch als Futter-Zusatzstoffe.

5. **Umhüllungs-Granulat**

| | |
|---|---|
| Wirkstoff oder Wirkstoffkombination | 3% |
| Polyäthylenglykol (MG 200) | 3% |
| Kaolin | 94% |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate. Solche Granulate eignen sich auch als Futter-Zusatzstoffe.

6. **Suspension-Konzentrat**

| | |
|---|---|
| Wirkstoff oder Wirkstoffkombination | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können. Suspensionskonzentrate können am Tier auch oral appliziert werden ("Drench").

7. **Komprimate**

| | | |
|---|---|---|
| I | Wirkstoff oder Wirkstoffkombination | 33,0% |
| | Methylcellulose | 0,8% |
| | Kieselsäure (hochdispers) | 0,8% |
| | Maisstärke | 8,4% |
| II | Milchzucker (kristallin) | 22,5% |
| | Maisstärke | 17,0% |
| | mikrokristalline Cellulose | 16,5% |
| | Magnesiumstearat | 1,0% |

Die Hilfsstoffe der Phase I werden mit dem Wirkstoff oder der Wirksto⁽ ·mbination unter Zusatz von 16,5 Teilen Wasser granuliert und dann mit den Hilfsstoffen der Phase II vermis· Die Mischung wird zu Tabletten oder Boli verpresst und getrocknet.

**Herstellungsbeispiel**

4,5 g (3-(3-Chlor-5-trifluormethyl-pyridyl-2-oxy)-4-methylanilin in 50 ml wasserfreiem Toluol werden mit 2,7 g 2,6-Difluorbenzoyliso-cyanat in 20 ml wasserfreiem Toluol versetzt. Nachdem die anfänglich exotherme Reaktion abgeklungen ist, wird das Gemisch über Nacht stehengelassen. Nach Filtration und Waschen mit Hexan erhält man in Form weisser Kristalle den N-3-(3-Chlor-5-trifluormethyl-pyridyl-2-oxy)-4-methylphenyl-N'-2,6-difluorbenzoylharnstoff vom Smp. 178-179° C (Verbindung Nr. 1).

Herstellung der Ausgangsverbindung:

Es werden 6,4 g Kaliumhydroxid in 30 ml Dimethylsulfoxid vorgelegt und zu diesem Gemisch eine Lösung von 15,3 g 2-Methyl-5-nitrophenol gelöst in 30 ml Dimethylsulfoxyd zugetropft. Nach Abklingen der exothermen Reaktion werden tropfenweise 19,9 g eines Gemisches aus 2-Fluor-3-chlor-5-trifluormethylpyridin (40 Gew.%) und 2,3-Dichlor-5-trifluor-methylpyridin (60 Gew.%) langsam zugesetzt. Nachdem auch diese exotherme Reaktion abgeklungen ist, wird das Reaktionsgemisch noch während zehn Stunden weiter gerührt. Dann wird das Gemisch auf Eiswasser gegossen und mit Dichlormethan extrahiert. Der Extrakt wird getrocknet und eingedampft. Der Rückstand wird mit einem Gemisch aus Dichlormethan und Hexan (Volumenverhältnis 35:15) über Kieselgel chromatographiert, wobei zu Beginn und am Ende des Chromatographierens reines Dichlormethan als Elutionsmittel verwendet wird. Das erhaltene Filtrat wird eingedampft und aus Hexan umkristallisiert. Man erhält das 3-(3-Chlor-5-trifluormethylpyridyl-2-oxy)-4-methyl-nitrobenzol als weisses kristallines Pulver vom Schmelzpunkt 93-94°C. Nun wird diese erhaltene Verbindung in Dioxan mit Raney-Nickel als Katalysator hydriert. Der Katalysator wird abfiltriert, die als Filtrat aufgefangene Reaktionslösung in Dichlormethan gelöst und über Kieselgel chromatographiert. Man erhält das 3-(3-Chlor-5-trifluormethyl-pyridyl-2-oxy)-4-methylanilin als weisses kristallines Pulver vom Schmelzpunkt 55° C.

Analog den vorstehend beschriebenen Arbeitsweisen oder nach einer der weiter oben beschriebenen Reaktionen wurden die folgenden Verbindungen der Formel I hergestellt:

Tabelle I

| Verbindung Nr. | $R_1$ | $R_2$ | $R_3$ | $R_4$ | Schmelzpunkt [°C] |
|---|---|---|---|---|---|
| 1 | F | F | $CH_3$ | Cl | 178-179 |
| 2 | F | F | H | H | 186-188 |
| 3 | Cl | H | H | H | 136-138 |
| 4 | F | F | Br | Cl | 198-200 |
| 5 | Cl | Cl | Br | Cl | 216-218 |
| 6 | Cl | H | Br | Cl | 213-214 |
| 7 | Cl | H | $CH_3$ | Cl | 162-164 |
| 8 | H | H | $CH_3$ | Cl | 228-229 |
| 9 | F | F | Br | H | 190-194 |
| 10 | $CH_3$ | H | $CH_3$ | H | 140-145 |
| 11 | Br | H | $CH_3$ | Cl | 164-165 |
| 12 | $CH_3$ | H | $CH_3$ | Cl | 177-178 |
| 13 | Cl | H | $CH_3$ | H | 143-144 |
| 14 | Br | Br | $CH_3$ | Cl | 219-222 |
| 15 | F | F | $CH_3$ | H | 188-189 |
| 16 | F | F | F | Cl | 174-177 |
| 17 | F | F | Cl | Cl | 185-189 |
| 18 | F | F | F | H | 183-184 |
| 19 | F | F | Cl | H | 185-188 |

Die Verbindungen Nr. 4, 16, 17, 18 und 19 sind als ektoparasitizide Wirkstoffe zur Bekämpfung von parasitären Insekten (Lucilia sericata, L. cuprina u.a.) und Acariden besonders bevorzugt.

Ein weiterer Gegenstand der Erfindung sind ektoparasitizide Mittel, die als Wirkstoff eine Verbindung der Formel I, insbesondere eine der Verbindungen Nr. 4, 16, 17, 18 oder 19 enthalten.

Ein weiterer Gegenstand der Erfindung sind Verfahren zur Bekämpfung von parasitären Insekten und Acarina mit Hilfe von Verbindungen der Formel I, insbesondere mit Hilfe einer der Verbindungen Nr. 4, 16, 17, 18 oder 19.

**Tabelle II**

Zwischenprodukte der Formel II zur Gewinnung von Verbindungen der Formel I

| Verb. | $R_3$ | $R_4$ | Smp. |
|---|---|---|---|
| A | $CH_3$ | Cl | 55° |
| B | H | H | 53-55° |
| C | Br | Cl | 79-81° |
| D | $CH_3$ | H | 64-66° |

| Verb. | $R_3$ | $R_4$ | Smp. |
|---|---|---|---|
| E | Cl | Cl | 82-85° |
| F | F | Cl | 49-52° |
| G | F | H | 54-57° |
| H | Cl | H | 63-65° |

**Biologische Beispiele**

**Beispiel 1**:

Wirkung gegen Musca domestica:

Je 50 g frisch zubereitetes CSMA-Nährsubstrat für Maden wurden in Becher eingewogen. Von einer 1 Gew.%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurde pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt. Verbindungen der Formel I zeigen im obigen Test eine vollständige Abtötung (Mortalität 100%).

**Beispiel 2**:

Wirkung gegen Aëdes aegypti:

Auf die Oberfläche von 150 ml Wasser, das sich in einem Behälter befindet, wurde so viel einer 0,1%igen acetonischen Lösung des Wirkstoffes pipettiert, dass Konzentrationen von je 800 und 400 ppm erhalten wurden. Nach Verdunsten des Acetons wurde der Behälter mit 30-40 2-tägigen Aëdes-Larven beschickt. Nach 1, 2 und 5 Tagen wurde die Mortalität geprüft.

Die Verbindungen Nr. 1 bis 19 der Formel I erzielten bereits nach einem Tag 100%ige Abtötung.

**Beispiel 3**:

Insektizide Frassgift-Wirkung:

Ca. 25 cm hohe eingetopfte Baumwollpflanzen wurden mit wässrigen Wirkstoffemulsionen besprüht, die den Wirkstoff in Konzentrationen von 12,5 und 100 ppm, enthielten. Nach dem Antrocknen des Sprühbelages wurden die Baumwollpflanzen mit Spodoptera littoralis- bzw. Heliothis virescens-Larven im dritten larvalen Stadium besiedelt. Der Versuch wurde bei 24°C und 60% relativer Luftfeuchtigkeit durchgeführt. Nach 120 Stunden wurde die %-Mortalität der Test-Insekten gegenüber unbehandelten Kontrollen ausgewertet.

Die nachstehende Tabelle zeigt Ergebnisse biologischer Prüfungen erfindungsgemässer Verbindungen auf der Basis des obigen biologischen Beispiels. Die Auswertung der Versuche erfolgte anhand der erhaltenen %-Mortalität unter Verwendung des folgenden Bewertungs-Index:

A: 80-100% Mortalität bei einer Konzentration von 12,5 ppm der geprüften Verbindung

B: 80-100% Mortalität bei einer Konzentration von 100 ppm der geprüften Verbindung

| Verbindung | pestizide Wirksamkeit | |
|---|---|---|
| | Spodoptera Larven | Heliothis Larven |
| 1 | A | A |
| 2 | A | A |
| 3 | A | A |
| 4 | A | B |
| 7 | B | A |

Gegen L-4-Larven des mexikanischen Bohnenkäfers (Epilachna varivestis) zeigen die Verbindungen Nr. 1-19 in Wirkstoff-Konzentrationen von 400 ppm 100% Abtötung nach 2 Tagen.

**Beispiel 4**

Reproduktionshemmende Wirkung bei Zecken

Frische, vollgesogene Weibchen der Zeckenart Boophilus microplus werden in Reihen zu 10 Tieren dorsal auf PVC-Platten aufgeklebt und mit einem Wattebausch überdeckt. Danach wird jede Reihe mit 10 ml der wässrigen Testlösung übergossen. Eine Stunde später wird der Wattebausch entfernt und die Zecken über Nacht bei 24°C getrocknet. Nach dem Trocknen werden die Zecken bei 28°C und 80% Luftfeuchtigkeit für 4 Wochen bis zum Ende der Eiablage und Beginn des Larvenschlupfes gehalten.

Jede Testsubstanz wird bei 5 Konzentrationen von 1000 bis 64 ppm (Verdünnungsfaktor 2) getestet. Die akarizide Wirkung zeigt sich entweder beim Weibchen als Mortalität oder Sterilität oder im Eigelege durch Blockieren der Embryogenese oder des Schlüpfaktes. Alle Substanzen werden gegen zwei verschiedene Zeckenstämme, den normal sensiblen Stamm YEERONGPILLY und den OP-resistenten Stamm BIARRA geprüft.

Die Aktivität einer Substanz wird auf Grund der tiefsten noch annähernd voll aktiven Konzentration bewertet (I R 100 ~ 100% Inhibition der Reproduktion).

Die Verbindungen Nr. 1 bis 19 der Formel I zeigen in diesem Test deutliche, reproduktionshemmende Wirkung bei Konzentrationen zwischen 64 und 500 ppm. Die Verbindungen Nr. 1,4,13 und 16 bis 19 sind bei 64 ppm voll wirksam.

**Beispiel 5**

Wirkung gegen Schmeissfliegen

Frisch abgelegte Eier der Schmeissfliegenart Lucilia sericate und L. cuprina werden in kleinen Portionen (30-50 Eier) in Reagenzgläser gegeben, in denen zuvor 4 ml Nährmedium mit 1 ml Testlösung in der für die Endkonzentration notwendigen Zwischenverdünnung der Aktivsubstanz vermischt worden sind. Nach Beimpfung des Kulturmediums werden die Testgläser mit einem Wattestopfen verschlossen und im Brutschrank bei 30°C über 4 Tage bebrütet. Im unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund und deutlich zurückgeblieben. Der Versuch wird simultan, mit 5 Konzentrationen von 100; 40; 16; 6,4 und 2,56 ppm durchgeführt. Die Wirksamkeit wird gemessen an der tiefsten noch voll wirksamen Konzentration (LC 100).

Die gute larvizide Wirkung der Verbindungen Nr. 1 bis 19 der Formel I zeigte sich in diesem Test durch 100%ige Mortalität der Larven nach spätestens 96 h bei Konzentrationen zwischen 40 und 2,56 ppm.

Die Verbindungen Nr, 2,3,4,7,13 und 15 bis 19 erzielten bei 6,4 ppm volle Wirkung.

**Patentansprüche** für die Vertragsstaaten BE CH DE FR IT LI NL

1. Verbindung der Formel I

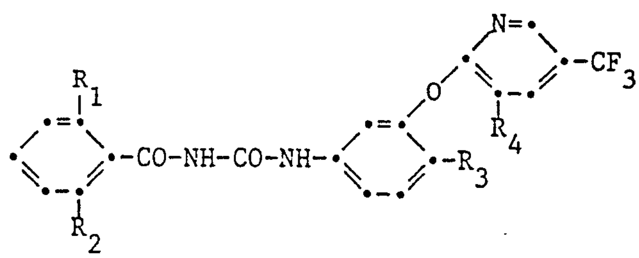

(I),

worin

$R_1$, $R_2$ und $R_3$ unabhängig voneinander Wasserstoff, Methyl, Fluor, Chlor oder Brom; und $R_4$ Wasserstoff oder Chlor bedeuten, mit der Massgabe $R_3$ Methyl, Fluor, Chlor oder Brom bedeutet, wenn $R_4$ die Bedeutung von Chlor hat.

2. Verbindung gemäss Anspruch 1, dadurch gekennzeichnet, dass $R_1$ Methyl, Fluor oder Chlor; $R_2$ Wasserstoff, Methyl, Fluor oder Chlor; $R_3$ Wasserstoff Methyl Fluor Chlor oder Brom; und $R_4$ Wasserstoff oder Chlor bedeuten.

3. Verbindung gemäss Anspruch 1 oder 2, dadurch gekennzeichnet, dass $R_1$ Fluor oder Chlor; $R_2$ Wasserstoff oder Fluor; $R_3$ Wasserstoff, Methyl oder Chlor und $R_4$ Wasserstoff oder Chlor bedeuten.

4 Verbindung gemäss Anspruch 1-3 dadurch gekennzeichnet, dass $R_3$ und/oder $R_4$ Wasserstoff bedeuten.

5. Verbindung gemäss Anspruch 1 bis 4, dadurch gekennzeichnet, dass $R_1$ und $R_2$ Fluor bedeuten.

6. Verbindung gemäss Anspruch 5, dadurch gekennzeichnet, dass $R_3$ Fluor oder Chlor und $R_4$ Wasserstoff oder Chlor bedeuten.

7. Verbindung gemäss Anspruch 5 der Formel

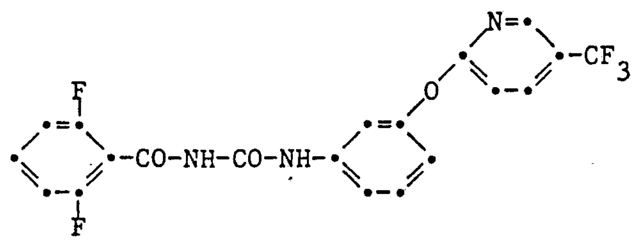

8. Verbindung gemäss Anspruch 5 der Formel

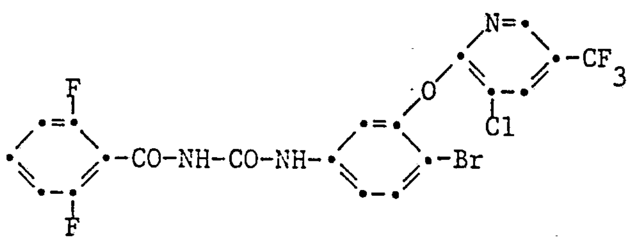

9. Verbindung gemäss Anspruch 5 der Formel

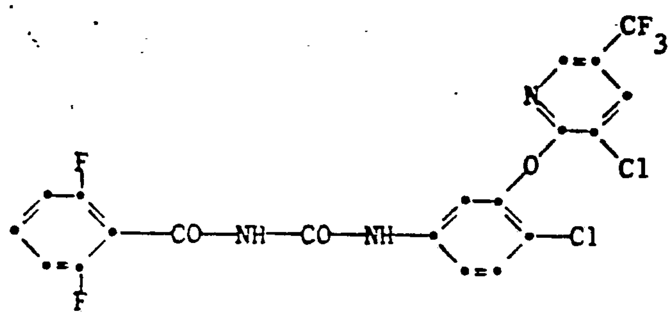

10. Verfahren zur Herstellung einer Verbindung gemäss den Ansprüchen 1 bis 9, dadurch gekennzeichnet, dass man

a) eine Verbindung der Formel II

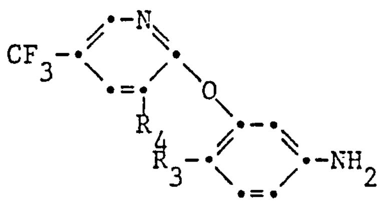

(II)

mit einer Verbindung der Formel III

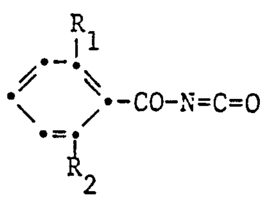

(III)

oder
b) eine Verbindung der Formel IV

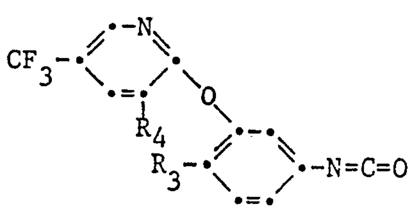

(IV)

mit einer Verbindung der Formel V

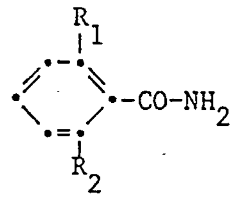

(V),

oder

c) eine Verbindung der Formel II mit einer Verbindung der Formel VI

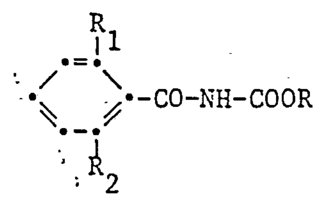

(VI)

umsetzt, wobei in den obigen Formeln II bis VI die Reste $R_1$ bis $R_4$ die in den Ansprüchen 1 bis 5 angegebenen Bedeutungen haben und R einen $C_1$-$C_8$-Alkylrest, der gegebenenfalls mit Halogen substituiert ist, bedeutet.

11. Mittel zur Bekämpfung von Insekten und Vetretern der Ordnung Acarina, welches als aktive Komponente eine Verbindung gemäss den Ansprüchen 1 bis 9 zusammen mit geeigneten Trägern und/oder anderen Zuschlagstoffen enthält.

12. Verbindung gemäss Anspruch 1 zur Bekämpfung von Insekten und Vertretern der Ordnung Acarina.

13. Verbindung gemäss Anspruch 12 zur Bekämpfung von tier- und pflanzenparasitären Vertretern der Ordnung Acarina.

14. Verbindungen der Formel I nach einem der Ansprüche 1 bis 9, zur Anwendung in einem Verfahren zur Bekämpfung von tierparasitären Vertretern der Ordnung Acarina.

15. Verbindungen der Formel II

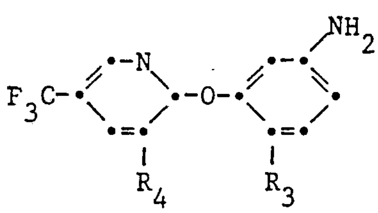

(II)

worin $R_4$ Wasserstoff und $R_3$ Wasserstoff, Methyl, Fluor, Chlor oder Brom bedeuten, oder worin $R_4$ Chlor und $R_3$ Methyl, Fluor, Chlor oder Brom darstellen.

**Patentansprüche** für den Vertragsstaat AT

1. Akarizides Mittel, enthaltend neben üblichen Formulierungshilfsstoffen als Wirkstoff eine Verbindung der Formel I

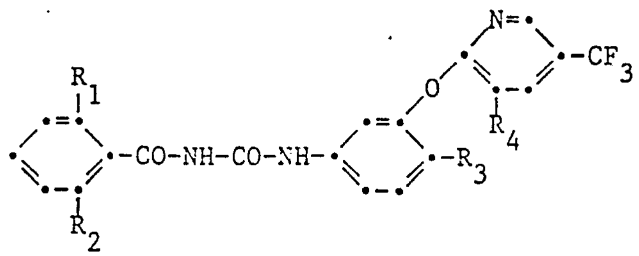

(I),

worin

$R_1$, $R_2$ und $R_3$ unabhängig voneinander Wasserstoff, Methyl, Fluor, Chlor oder Brom; und $R_4$ Wasserstoff oder Chlor bedeuten, mit der Massgabe $R_3$ Methyl Fluor, Chlor oder Brom bedeutet, wenn $R_4$ die Bedeutung von Chlor hat.

2. Mittel nach Anspruch 1, enthaltend als Wirkstoff eine Verbindung der Formel I, worin $R_1$ Methyl, Fluor oder Chlor; $R_2$ Wasserstoff, Methyl, Fluor oder Chlor; $R_3$ Wasserstoff, Methyl, Fluor, Chlor oder Brom und $R_4$ Wasserstoff oder Chlor bedeuten.

3. Mittel nach einem der Ansprüche 1 oder 2, enthaltend als Wirkstoff eine Verbindung der Formel I, worin $R_1$ Fluor oder Chlor; $R_2$ Wasserstoff oder Fluor; $R_3$ Wasserstoff, Methyl oder Chlor und $R_4$ Wasserstoff oder Chlor bedeuten.

4. Mittel nach einem der Ansprüche 1 bis 3, enthaltend als Wirkstoff eine Verbindung der Formel I, worin $R_3$ und/oder $R_4$ Wasserstoff bedeuten.

5. Mittel nach einem der Ansprüche 1 bis 4, enthaltend als Wirkstoff eine Verbindung der Formel I, worin $R_1$ und $R_2$ Fluor bedeuten.

6. Mittel nach Anspruch 5, enthaltend als Wirkstoff eine Verbindung der Formel I, worin $R_3$ Fluor oder Chlor und $R_4$ Wasserstoff oder Chlor bedeuten.

7. Mittel nach Anspruch 5, enthaltend als Wirkstoff die Verbindung der Formel

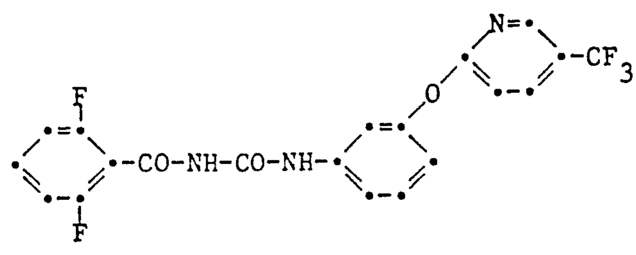

.

8. Mittel nach Anspruch 5, enthaltend als Wirkstoff die Verbindung der Formel

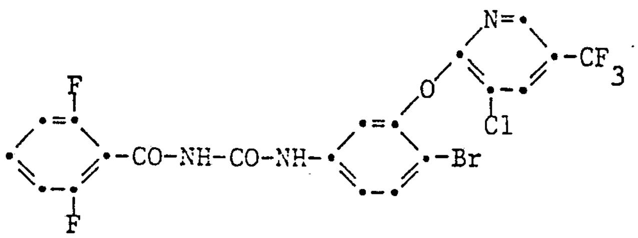

9. Mittel nach Anspruch 5, enthaltend als Wirkstoff die Verbindung der Formel

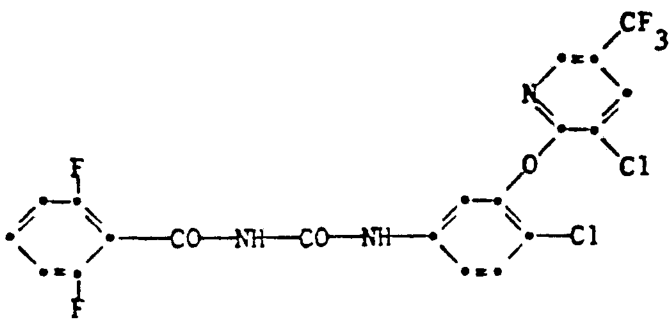

10. Verfahren zur Herstellung einer Verbindung der Formel I gemäss den Ansprüchen 1 bis 9, dadurch gekennzeichnet, dass man
a) eine Verbindung der Formel II

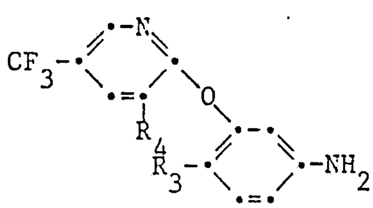

(II)

mit einer Verbindung der Formel III

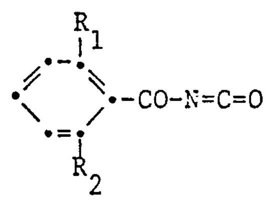

(III)

oder
b) eine Verbindung der Formel IV

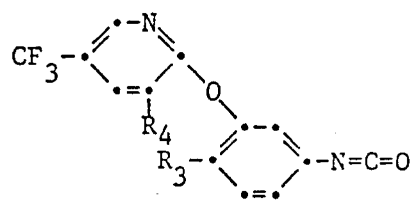

(IV)

mit einer Verbindung der Formel V

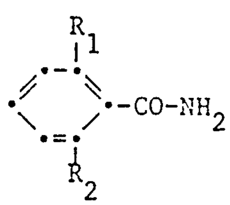

(V),

oder
c) eine Verbindung der Formel II mit einer Verbindung der Formel VI

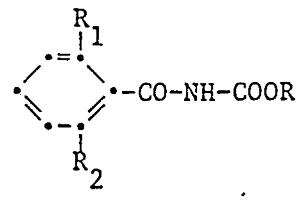

(VI)

umsetzt, wobei in den obigen Formeln II bis VI die Reste $R_1$ bis $R_4$ die in den Ansprüchen 1 bis 5 angegebenen Bedeutungen haben und R einen $C_1$-$C_8$-Alkylrest, der gegebenenfalls mit Halogen substituiert ist, bedeutet.

11. Verbindung der Formel I gemäss Anspruch 1 zur Bekämpfung von Insekten und Vertretern der Ordnung Acarina.

12. Verbindung gemäss Anspruch 11 zur Bekämpfung von tier- und pflanzenparasitären Vertretern der Ordnung Acarina.

**Claims** for the Contracting States: BE, CH, DE, FR, IT, LI, NL.

1. A compound of the formula

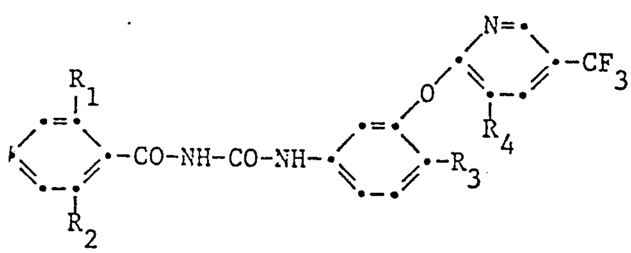

(I)

wherein
$R_1$, $R_2$ and $R_3$ are each independently hydrogen, methyl, fluorine, chlorine or bromine, and
$R_4$ is hydrogen or chlorine, with the proviso that $R_3$ is methyl, fluorine, chlorine or bromine when $R_4$ is chlorine.

2. A compound according to claim 1, wherein $R_1$ is methyl, fluorine or chlorine, $R_2$ is hydrogen, methyl, fluorine or chlorine, $R_3$ is hydrogen, methyl, fluorine, chlorine or bromine, and $R_4$ is hydrogen or chlorine.

3. A compound according to either of claims 1 or 2, wherein $R_1$ is fluorine or chlorine, $R_2$ is hydrogen or fluorine, $R_3$ is hydrogen, methyl or chlorine, and $R_4$ is hydrogen or chlorine.

4. A compound according to any one of claims 1 to 3, wherein $R_3$ and/or $R_4$ are hydrogen.

5. A compound according to any one of claims 1 to 4, wherein $R_1$ and $R_2$ are fluorine.

6. A compound according to claim 5, wherein $R_3$ is fluorine or chlorine, and $R_4$ is hydrogen or chlorine.

7. The compound according to claim 5 of the formula

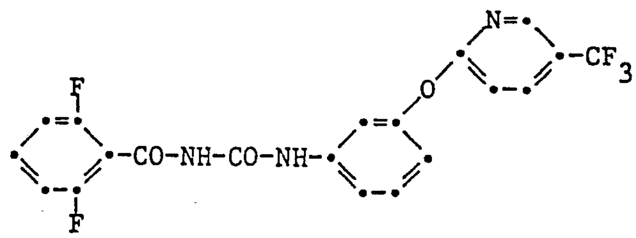

8. The compound according to claim 5 of the formula

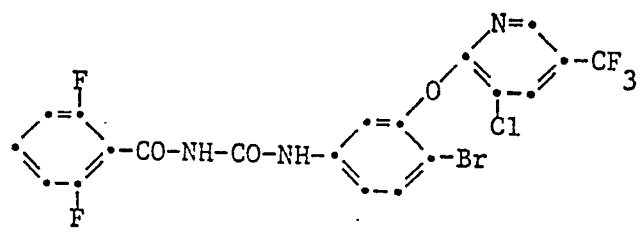

9. The compound according to claim 5 of the formula

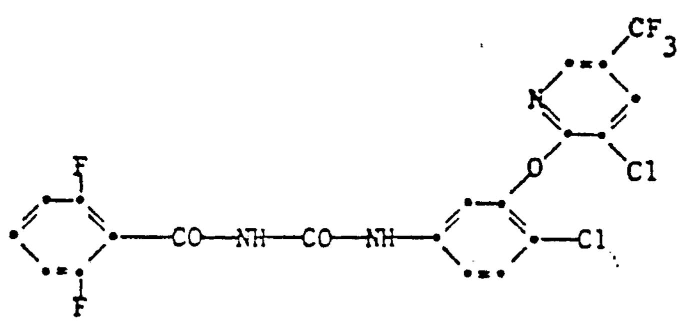

10. A process for the preparation of a compound according to any one of claims 1 to 9, which process comprises reacting
a) a compound of the formula II

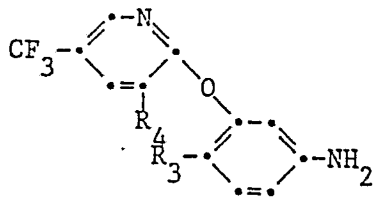

(II)

with a compound of the formula III

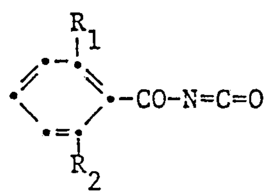

(III);

or
b) a compound of the formula IV

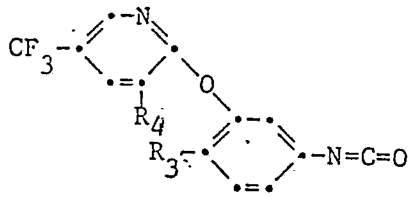

(IV)

with a compound of the formula V

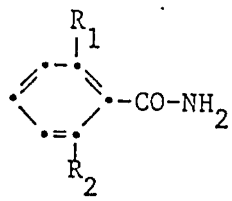

(V);

or

c) a compound of the formula II with a compound of the formula VI

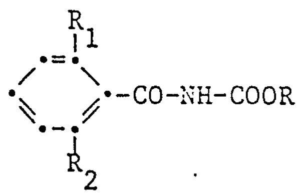

(VI);

$R_1$ to $R_4$ in the above formulae II to VI being as defined in any one of claims 1 to 5, and R being a $C_1$-$C_8$-alkyl group which is unsubstituted or substituted by halogen.

11. A composition for controlling insects and representatives of the order Acarina, which composition contains as active ingredient a compound according to any one of claims 1 to 9, together with suitable carriers and/or other additives.

12. A compound I according to claim 1 for controlling insects and representatives of the order Acarina.

13. A compound according to claim 12 for controlling representatives of the order Acarina which are parasitic on animals and plants.

14. A compound of the formula I according to any one of claims 1 to 9 for use in a method of controlling representatives of the order Acarina which are parasitic on animals.

15. A compound of the formula II

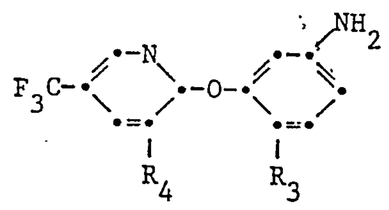

(II)

wherein $R_4$ is hydrogen, and $R_3$ is hydrogen, methyl, fluorine, chlorine or bromine; or wherein $R_4$ is chlorine, and $R_3$ is methyl, fluorine, chlorine or bromine.

**Claims** for the Contracting State: AT

1. An acaricidal composition containing as active ingredient a compound of the formula I

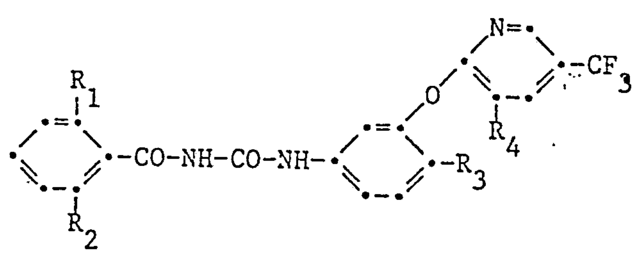

(I)

wherein
$R_1$, $R_2$ and $R_3$ are each independently hydrogen, methyl, fluorine, chlorine or bromine, and
$R_4$ is hydrogen or chlorine, with the proviso that $R_3$ is methyl, fluorine, chlorine or bromine when $R_4$ is chlorine,
together with customary formulation assistants.

2. A composition according to claim 1, containing as active ingredient a compound of the formula I, wherein $R_1$ is methyl, fluorine or chlorine, $R_2$ is hydrogen, methyl, fluorine or chlorine, $R_3$ is hydrogen, methyl, fluorine, chlorine or bromine, and $R_4$ is hydrogen or chlorine.

3. A composition according to either of claims 1 or 2, containing as active ingredient a compound of the formula I, wherein $R_1$ is fluorine or chlorine, $R_2$ is hydrogen or fluorine, $R_3$ is hydrogen, methyl or chlorine, and $R_4$ is hydrogen or chlorine.

4. A composition according to any one of claims 1 to 3, containing as active ingredient a compound of the formula I, wherein $R_3$ and/or $R_4$ are hydrogen.

5. A composition according to any one of claims 1 to 4, containing as active ingredient a compound of the formula I, wherein $R_1$ and $R_2$ are fluorine.

6. A composition according to claim 5, containing as active ingredient a compound of the formula I, wherein $R_3$ is fluorine or chlorine, and $R_4$ is hydrogen or chlorine.

7. A composition according to claim 5, containing as active ingredient the compound of the formula

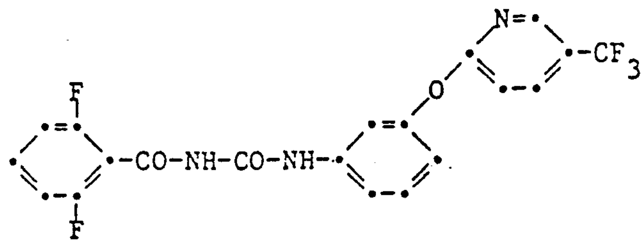

.

8. A composition according to claim 5, containing as active ingredient the compound of the formula

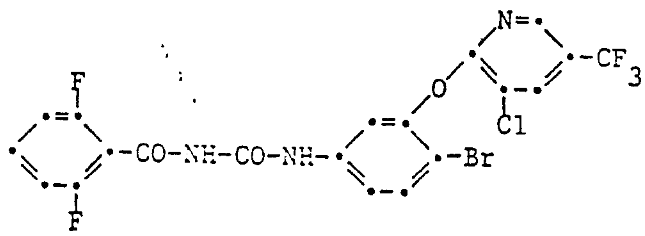

.

9. A composition according to claim 5, containing as active ingredient the compound of the formula

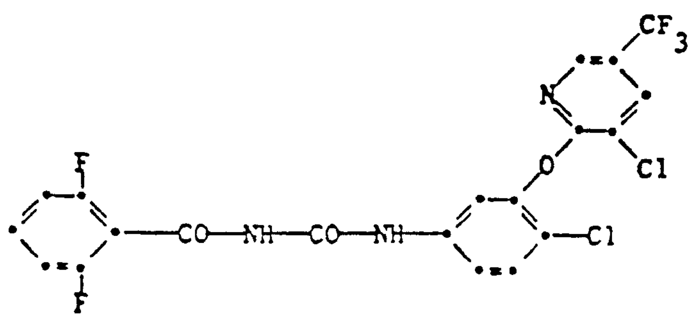

.

10. A process for the preparation of a compound of the formula I according to any one of claims 1 to 9, which process comprises reacting
a) a compound of the formula II

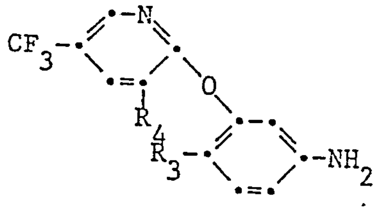

(II)

with a compound of the formula III

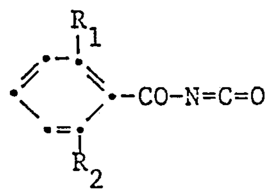

(III);

or
b) a compound of the formula IV

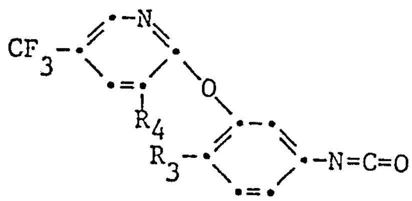

(IV)

with a compound of the formula V

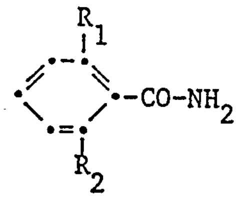

(V);

or

c) a compound of the formula II with a compound of the formula VI

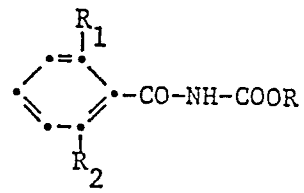

(VI);

$R_1$ to $R_4$ in the above formulae II to VI being as defined in any one of claims 1 to 5, and R being a $C_1$-$C_8$-alkyl group which is unsubstituted or substituted by halogen.

11. A compound of the formula I according to claim 1 for controlling insects and representatives of the order Acarina.

12. A compound according to claim 11 for controlling representatives of the order Acarina which are parasitic on animals and plants.

**Revendications** pour les Etats contractants: BE, CH, DE, FR, IT, LI, NL

1. Composé de formule I

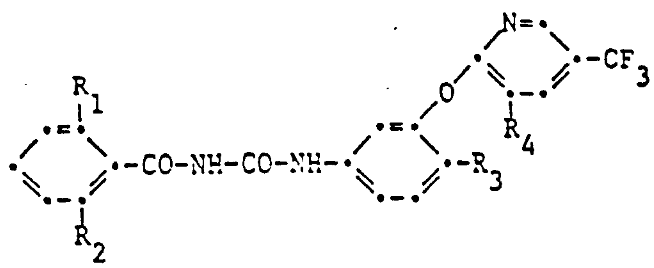

(I),

dans laquelle

$R_1$, $R_2$ et $R_3$ représentent chacun, indépendamment les uns des autres, l'hydrogène, un groupe méthyle, le fluor, le chlore ou le brome; et $R_4$ représente l'hydrogène ou le chlore, avec la restriction que $R_3$ représente un groupe méthyle, le fluor, le chlore ou le brome lorsque $R_4$ représente le chlore.

2. Composé selon la revendication 1, caractérisé en ce que $R_1$ représente un groupe méthyle, le fluor ou le chlore; $R_2$ représente l'hydrogène, un groupe méthyle, le fluor ou le chlore; $R_3$ représente l'hydrogène, un groupe méthyle, le fluor, le chlore ou le brome; et $R_4$ représente l'hydrogène ou le chlore.

3. Composé selon la revendication 1 ou 2, caractérisé en ce que $R_1$ représente le fluor ou le chlore; $R_2$ représente l'hydrogène ou le fluor; $R_3$ représente l'hydrogène, un groupe méthyle ou le chlore et $R_4$ l'hydrogène ou le chlore.

4. Composé selon les revendications 1 à 3, caractérisé en ce que $R_3$ et/ou $R_4$ représentent l'hydrogène.

5. Composé selon les revendications 1 à 4, caractérisé en ce que $R_1$ et $R_2$ représentent le fluor.

6. Composé selon la revendication 5, caractérisé en ce que $R_3$ représente le fluor ou le chlore et $R_4$ l'hydrogène ou le chlore.

7. Composé selon la revendication 5, de formule

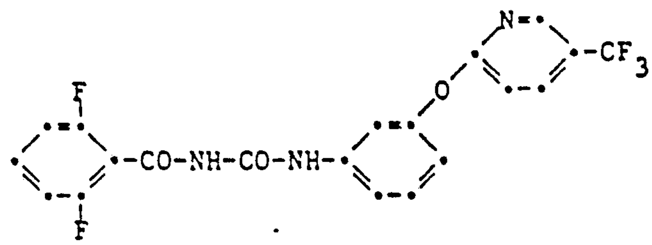

8. Composé selon la revendication 5, de formule

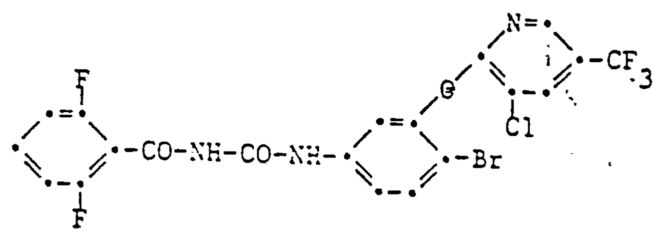

9. Composé selon la revendication 5 de formule

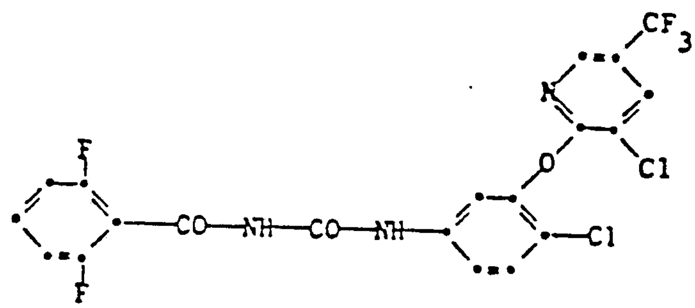

10. Procédé de préparation d'un composé selon les revendications 1 à 9, caractérisé en ce que:
a) on fait réagir un composé de formule II

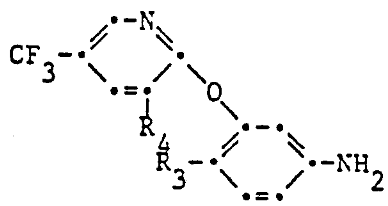

(II)

avec un compose de formule III

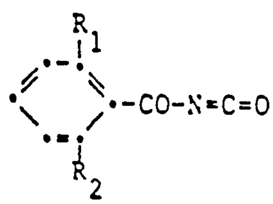

(III)

ou bien
b) un composé de formule IV

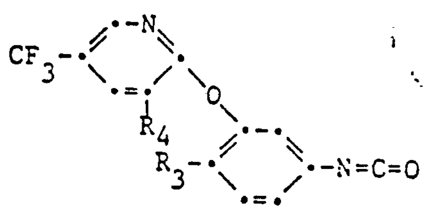

(IV)

avec un composé de formule V

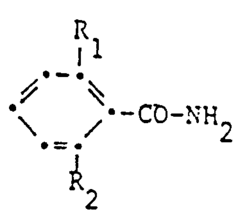

(V)

ou bien
c) un compose de formule II avec un composé de formule VI

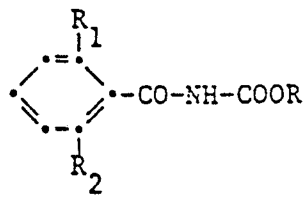

(VI)

les symboles $R_1$ à $R_4$ ayant dans les formules II à VI ci-dessus les significations indiquées dans les revendications 1 à 5 et R représentant un groupe alkyle en $C_1$-$C_8$ éventuellement substitué par des halogènes.

11. Produit pour combattre les insectes et les représentants de l'ordre des acariens, qui contient en tant que composant actif un composé selon les revendications 1 à 9 avec des véhicules et/ou d'autres additifs appropriés.

12. Composé selon la revendication 1, pour la lutte contre les insectes et les représentants de l'ordre des acariens.

13. Composé selon la revendication 12, pour la lutte contre les représentants de l'ordre des acariens parasitant les animaux et les végétaux.

14. Composés de formule I selon l'une des revendications 1 à 9, pour l'utilisation dans un procédé pour la lutte contre les représentants de l'ordre des acariens parasitant les animaux.

15. Composés de formule II

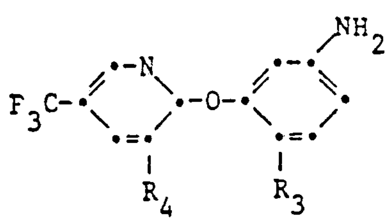

(II)

dans laquelle $R_4$ représente l'hydrogène et $R_3$ l'hydrogène, un groupe méthyle, le fluor, le chlore ou le brome, ou bien dans laquelle $R_4$ représente le chlore et $R_3$ un groupe méthyle, le fluor, le chlore ou le brome.

**Revendications** pour l'Etat contractant: AT

1. Produit acaricide contenant en tant que substance active, avec des produits auxiliaires de formulation usuels, un composé de formule I

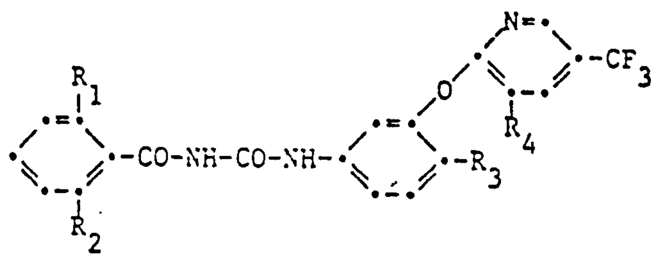

(I),

dans laquelle

$R_1$, $R_2$ et $R_3$ représentent chacun, indépendamment les uns des autres, l'hydrogène, un groupe méthyle, le fluor, le chlore ou le brome; et $R_4$ représente l'hydrogène ou le chlore, avec la restriction que $R_3$ représente un groupe méthyle, le fluor, le chlore ou le brome lorsque $R_4$ représente le chlore.

2. Produit selon la revendication 1, contenant en tant que substance active un composé de formule I dans laquelle $R_1$ représente un groupe méthyle, le fluor ou le chlore; $R_2$ représente l'hydrogène, un groupe méthyle, le fluor ou le chlore; $R_3$ représente l'hydrogène, un groupe methyle, le fluor, le chlore ou le brome et $R_4$ représente l'hydrogène ou le chlore.

3. Produit selon l'une des revendications 1 ou 2, contenant en tant que substance active un composé de formule I dans laquelle $R_1$ représente le fluor ou le chlore; $R_2$ représente l'hydrogène ou le fluor; $R_3$ représente l'hydrogène, un groupe méthyle ou le chlore et $R_4$ l'hydrogène ou le chlore.

4. Produit selon l'une des revendications 1 à 3, contenant en tant que substance active un composé de formule I dans laquelle $R_3$ et/ou $R_4$ représentent l'hydrogène.

5. Produit selon l'une des revendications 1 à 4, contenant en tant que substance active un composé de formule I dans laquelle $R_1$ et $R_2$ représentent le fluor.

6. Produit selon la revendication 5, contenant en tant que substance active un composé de formule I dans laquelle $R_3$ représente le fluor ou le chlore et $R_4$ l'hydrogène ou le chlore.

7. Produit selon la revendication 5, contenant en tant que substance active le composé de formule

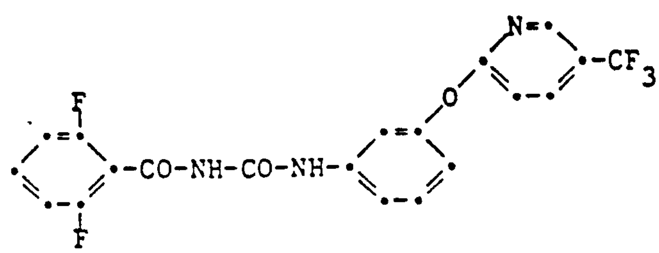

8. Produit selon la revendication 5, contenant en tant que substance active le composé de formule

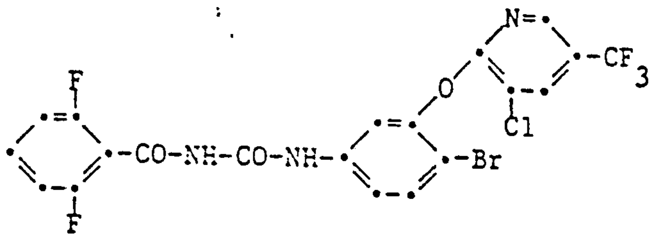

9. Produit selon la revendication 5, contenant en tant que substance active le composé de formule

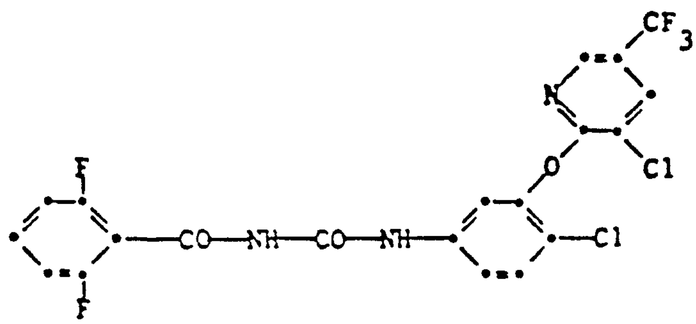

10. Procédé de préparation d'un composé de formule I selon les revendications 1 à 9, caractérisé en ce que a) on fait réagir un composé de formule II

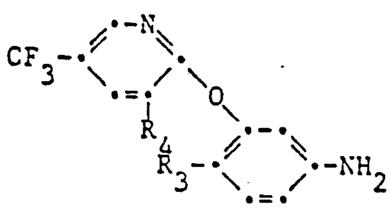

(II)

avec un composé de formule III

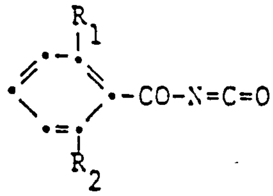

(III)

ou bien

b) on fait réagir un composé de formule IV

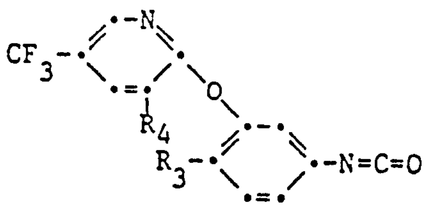

(IV)

avec un composé de formule V

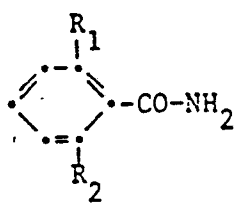

(V)

ou bien

c) on fait réagir un composé de formule II avec un composé de formule VI

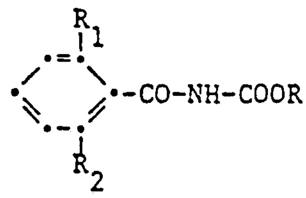

(VI)

les symboles $R_1$ à $R_4$ ayant dans les formules II à VI ci-dessus les significations indiquées dans les revendications 1 à 5 et R représentant un groupe alkyle en $C_1$-$C_8$ éventuellement substitué par des halogènes.

11. Composé de formule I selon la revendication 1, pour la lutte contre les insectes et les représentants de l'ordre des acariens.

12. Composé selon la revendication 11, pour la lutte contre les représentants de l'ordre des acariens parasitant les animaux et les végétaux.